# EUROPEAN PATENT APPLICATION

(11) **EP 3 067 381 A1**
(43) Date of publication of application: **14.09.2016**
(21) Application number: 15158791.2
(22) Date of filing: 12.03.2015
(51) Int. Cl.: C08G 65/24, C07C 43/02, C08G 83/00, C08F 220/28, C09D 133/06, C08G 65/26, C09D 171/02

(54) **Coated silicon surfaces, their manufacture and application**

(71) Applicant: BASF SE, 67056 Ludwigshafen am Rhein (DE)
(72) Inventor: Lindner, Jean-Pierre Berkan, 68165 Mannheim (DE); Bruchmann, Bernd, 67251 Freinsheim (DE); Xiaoyu, Sun, 8057 Zürich (SZ); Schlüter, A.Dieter, 8032 Zürüch (SZ)
(74) Representative: BASF IP Association

(57) **Abstract**

Process for coating silicon surfaces comprising the step of treating said silicon surface with at least one (co)polymer A, comprising in copolymerized form
(A) at least one comonomer bearing a structural unit according to the general formula (I) 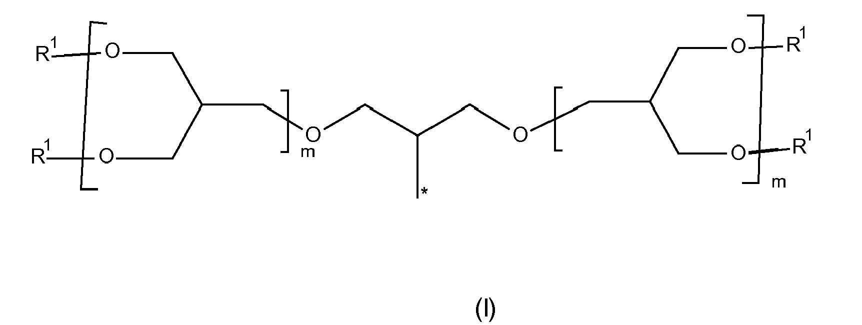 wherein
R¹ is 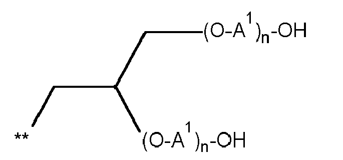 m is independently from each other selected from zero to 6,
n is independently from each other selected from zero to 200,
A¹ are different or identical and selected from C₂-C₁₀-alkylene, C₆-C₁₀-arylene and C₇-C₁₀-aralkylene,

(B) optionally, at least one ethylenically unsaturated carboxylic acid,
(C) optionally, at least one further monomer.

## Description

The present invention relates to a process for coating silicon surfaces comprising the step of treating said silicon surface with at least one (co)polymer A, comprising in copolymerized form
(A) at least one comonomer bearing a structural unit according to the general formula (I) 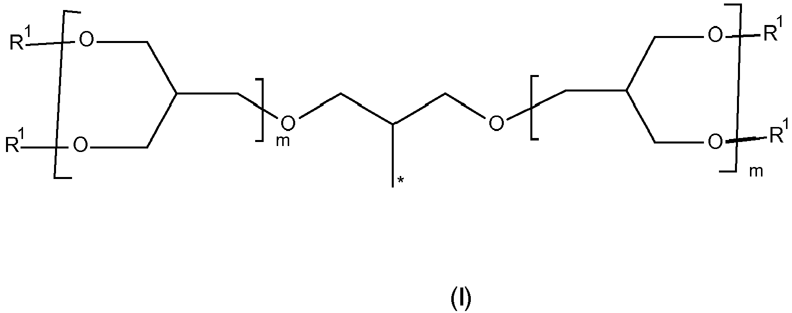 wherein
   - R¹: is

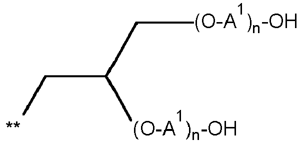

   - m: is independently from each other selected from zero to 6,
   - n: is independently from each other selected from zero to 200,
   - A¹: are different or identical and selected from C₂-C₁₀-alkylene, C₆-C₁₀-arylene and C₇-C₁₀-aralkylene,
(B) optionally, at least one ethylenically unsaturated carboxylic acid,
(C) optionally, at least one further monomer.

Furthermore, the present invention relates to coated silicon surfaces. Furthermore, the present invention relates to applications and uses of inventive coated silicon surfaces, in particular as part of or for silicon wafers. In addition, the present invention relates to (co)polymers A and a process of their manufacture.

Silicon surfaces play a significant role for numerous applications, for examples in the form of wafers or silicon oxide coated polymer films. In several applications it is necessary to have a pure silicon surface. For various applications, however, it is desirable to attach something to the silicon surface. There may be a separate layer or a device to be attached to the silicon surface. Such attached layer or device is expected to not be removed or peel off under the influence of water including humidity. Many coatings, however, either cause a mechanical impact on the surface, or the stableness of such attachment leaves room for improvement.

In other applications, for example food packaging, aluminoxide ("AlOx") or silica ("SiOx") coated polymer films are being used, such polymers being selected from polyethylene terephthalate (PET), polyethylene (PE), polypropylene (PP), or polyamide ("PA"). In order to laminate an additional film on such coated film a so-called primer needs to be found. Such primer should not damage the AlOx- or SiOx-coated film.

It was therefore an objective of the present invention to provide a process that can overcome the disadvantages discussed above. It was furthermore an objective of the present invention to provide coated silicon surfaces that can overcome the disadvantages discussed above, and it was an objective to provide applications of such surfaces.

Accordingly, the process defined at the outset has been found, hereinafter also being referred to as inventive coating process or coating process according to the present invention.
The inventive coating process starts with a silicon surface. Said silicon may be pure or ultrapure, for example with a purity of 99.9999999% ("9N") or more, useful for semiconductor devices, or less pure, for example 99.9999 to 99.999999%, so called high-purity polycrystalline silicon. In other embodiments, metallurgical grade silicon may be used, with a purity of 98% or more but less than "6N". The silicon may be covered by a monomolecular layer of SiO₂.

The above silicon surface may be rough, may exhibit dents visible with naked eye, or appear even to the naked eye. Preferably, said silicon surface appears even to the naked eye.

Said silicon surface may constitute a part of a silicon wafer. In other embodiments of the present invention, such silicon may constitute a SiOx-coated polymer film, polymer being selected from PET, PE, PP, and PA.

The inventive coating process may comprise one or more steps. Optional steps may refer to the preparation of the silicon surface, or adding one or more additives copolymer A if applied in solution.

The inventive process comprises the step of treating said silicon surface with at least one (co)polymer A. Said (co)polymer A may be a homopolymer or a copolymer. It contains in polymerized form
(A) one or more comonomers that bear a structural unit according to general formula (I), hereinafter also being referred to as (A), and, optionally,
(B) at least one at least one ethylenically unsaturated carboxylic acid (B), hereinafter also being referred to as comonomer (B) or ethylenically unsaturated carboxylic acid (B), said ethylenically unsaturated carboxylic acid (B) being different from comonomer (A), and, optionally,
(C) at least one further comonomer, hereinafter also being referred to as comonomer (C).

In one embodiment of the present invention comonomer (A) bears a polymerizable group selected from vinyl, allyl, (meth)acryloyl, maleic acid and styryl groups.

The comonomers (A) to (C) shall be explained in more detail further down below.

Said silicon surface may be treated with (co)polymer A in bulk or in solution, preferably in solution. As solvents, polar solvents like ethanol or ethylene glycol are possible. It is even more preferred to treat said silicon surface with (co)polymer A in aqueous solution. The term "aqueous solution" as used in the context with the instant invention shall not only include solutions in pure water but also in mixtures of water with at least one organic solvent that is miscible with water, wherein in such mixtures the percentage by volume of water is at least 50%, preferably 75 to 99%. The term aqueous solutions shall also include aqueous solutions of an acid or a base, for example acetic acid, formic acid, aqueous potassium hydroxide solution and aqueous sodium hydroxide solution.

In one embodiment of the present invention, said solution and preferably such aqueous solution contains in the range of from 0.5 to 10 g/l (co)polymer A, preferably up to 5 g/l (co)polymer (A) and even more preferably 0.6 to 1 g/l (co)polymer A. Solutions with a higher concentration may be hard to handle. Solutions with a lower concentration hardly contain (co)polymer, and removal of the high percentage of solvent or water may render the process less favorable from an economic standpoint.

In one embodiment of the present invention, aqueous solutions of (co)polymer A may include one or more auxiliary ingredients. The term "auxiliary ingredients" as used in the context of the present invention refers to ingredients other than (co)polymer A, water, organic solvents and the above acids and bases. Examples of auxiliary ingredients are surfactants, for example non-ionic surfactants such as, but not limited to alkoxylated and in particular propoxylated and ethoxylated fatty alcohols.

In an alternative embodiment of the present invention, aqueous solutions of (co)polymer A do not contain any auxiliary ingredient.

In one embodiment of the present invention, solution containing (co)polymer A is applied to said silicon surface by dipping the silicon surface into said solution. The time of residence of said silicon surface in said solution may be in the range of from 5 minutes to 6 hours.

In other embodiment, said solution is sprayed on the silicon surface or applied by air brush. In other embodiments, solution of (co)polymer A is applied with a doctor blade or a spread knife or a squeegee.

In embodiments wherein said silicon surface is treated with (co)polymer in bulk, (co)polymer A may be applied in melt.

In one embodiment of the present invention, the inventive coating process is performed at a temperature in the range of from ambient temperature to 75°C. In embodiments wherein a solution of (co)polymer A is applied the temperature refers to the temperature of the respective solution.

The inventive coating process may be performed at normal pressure.

After the application of said (co)polymer A to said silicon surface, solvent or preferably water - if applicable - is removed. Said removal may be performed by drying, for example drying in a drying chamber, drying under vacuum, or freeze-drying, or by blowing air with a temperature in the range of from 20 to 80°C over said surface.

After having performed the inventive coating process a coated silicon surface is obtained that also constitutes an aspect of the present invention, silicon surfaces being selected from silicon wafers and SiOx-coated polymer films. Said coated silicon surface may be fixed to polymers films such as, but not limited to films made from polyethylene, polypropylene, polyamide, and polyester such as, but not limited to polyethylenterephthalate. Other examples are glues and printed images from printing inks.

Such coated silicon surface may be fixed polymer foils showing a better adhesion leading to an overall more stable foil, which can be used for application such as, but not limited to food packaging.

A further aspect of the present invention relates to silicon surfaces coated with at least one (co)polymer A, comprising in copolymerized form
(A) at least one comonomer bearing a structural unit according to the general formula (I) 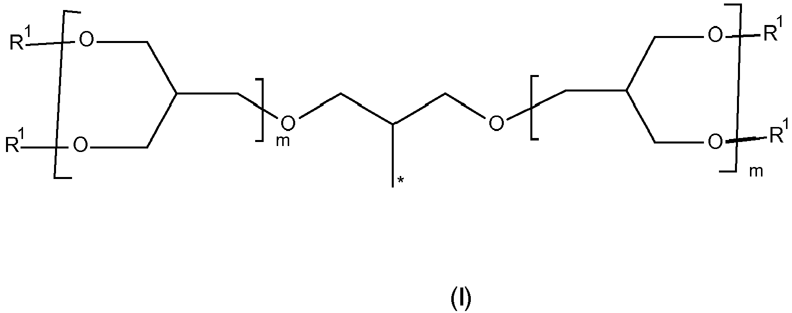 Wherein
   - R¹: is

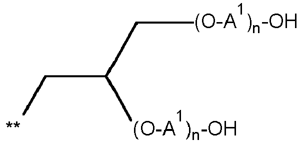

   - m: is independently from each other selected from zero to 6,
   - n: is independently from each other selected from zero to 200,
   - A¹: are different or identical and selected from C₂-C₁₀-alkylene, C₆-C₁₀-arylene and C₇-C₁₀-aralkylene,
(B) optionally, at least one ethylenically unsaturated carboxylic acid,
(C) optionally, at least one further monomer.

In the context of the present invention, said coated silicon surfaces may also be referred to as inventive coated surfaces.

The term silicon and the variables n, m and A¹ have been defined above.

Said (co)polymer A may be a homopolymer or a copolymer. It contains in polymerized form - one or more comonomers (A), and it may optionally contain at least one at least one ethylenically unsaturated carboxylic acid (B), ethylenically unsaturated carboxylic acid being different from comonomer (A). (co)polymer A may comprise at least one further comonomer (C). The comonomers (A) to (C) shall be explained in more detail further down below.

Inventive coated surfaces comprise a layer of (co)polymer A. said layer may have gaps or-preferably - be continuous.

Such silicon surface constitutes a part of a silicon wafer. In other embodiments of the present invention, such silicon surface may constitute a SiOx-coated polymer film, polymer being selected from PET, PE, PP, and PA.

In one embodiment of the present invention, the layer of (co)polymer A has a thickness in the range may be in the range of from 1 to 100 nm, preferably 3 to 10 nm. The thickness may be determined by variable angle spectroscopic ellipsometry ("VASE"). Preferably, the layer of (co)polymer A has about the same thickness throughout the whole layer. This means that the thickness varies by 10% or less.

Inventive coated silicon surfaces exhibit great mechanical stability. It is hard to remove the coating from the silicon with the help of solvents or the like, and the tendency to peel off is low. Inventive coated surfaces may advantageously be used as part of laminating foil for food packaging. In addition, a coating made according to the present invention may advantageously be used as primer. Additional layers of materials that would not stick well to silicon directly may be applied on said coating. Examples are polymers such as polyethylene and polypropylene.

Another aspect of the present invention relates to (co)polymers A and to a method of their manufacture, hereinafter also being referred to as inventive (co)polymers A and as inventive (co)polymer synthesis, respectively.

Inventive (co)polymers A may be selected from homopolymers and copolymers. Copolymers may contain at least two different comonomers (A) in polymerized form but neither comonomer (B) nor (C), or they may contain at least one comonomer (A) and at least one comonomer (B) or at least one comonomer (C) in polymerized form.

(Co)polymer A comprises - in copolymerized form - at least one comonomer (A). Comonomer
(A) bears a structural unit according to the general formula (I) 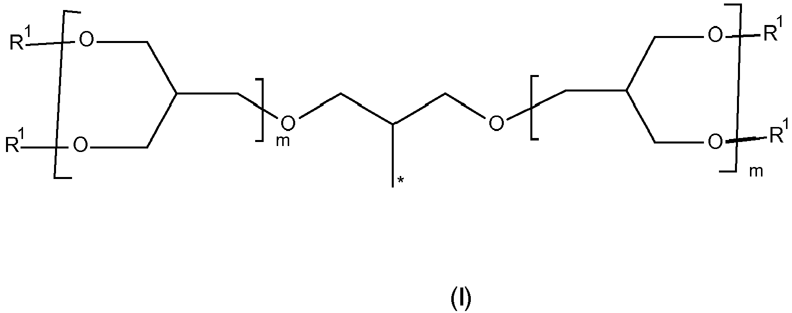 wherein
   - R¹: is

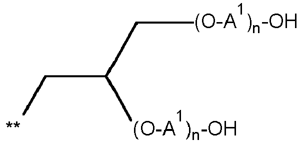

   - m: is independently from each other selected from zero to 6, preferably from zero to 3, and even more preferably all m are identical and equal zero,
   - A¹: is different or preferably identical and selected from C₆-C₁₀-arylene, for example orthophenylene, metaphenylene, paraphenylene, 1,6-naphthylene, 1,7-naphthylene, 2,6-naphthylene, 2,7-naphthylene or 1,8-naphthylene,
   C₇-C₁₀-aralkylene, such as, for example, -CH(C₆H₅)- or -CH₂-CH(C₆H₅)-, and in particular from C₂-C₁₀-alkylene, substituted or preferably unsubstituted, for example -CH₂-, -CH(CH₃)-, -CH(C₂H₅)-, -C(CH₃)₂-, -CH₂-CH₂-, -CH(CH₃)-CH₂-, -CH₂-CH(CH₃)-, -CH(C₂H₅)-CH₂-, -CH₂-CH(C₂H₅)-, -(CH₂)₃-, -(CH₂)₄-, -(CH₂)₅-, -(CH₂)₆-, -(CH₂)₈-, -(CH₂)₁₀-, preferably -CH₂-CH₂-, -CH(CH₃)-CH₂-, -CH₂-CH(CH₃)-, very particularly preferably -CH₂-CH₂- ("EO"),
   - n: is independently from each other selected from zero to 200, preferably zero to 150, particularly preferably up to 50. In a preferred embodiment, all n are equal. Even more preferably, n = zero. In that embodiment, comonomer (A) bears a structural unit according to the formula (I a)

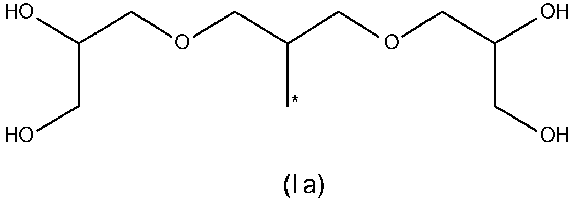

In another embodiment, comonomer (A) is a mixture of two comonomers, one bearing a structural unit (I a) and the other bearing a structural unit (I b)

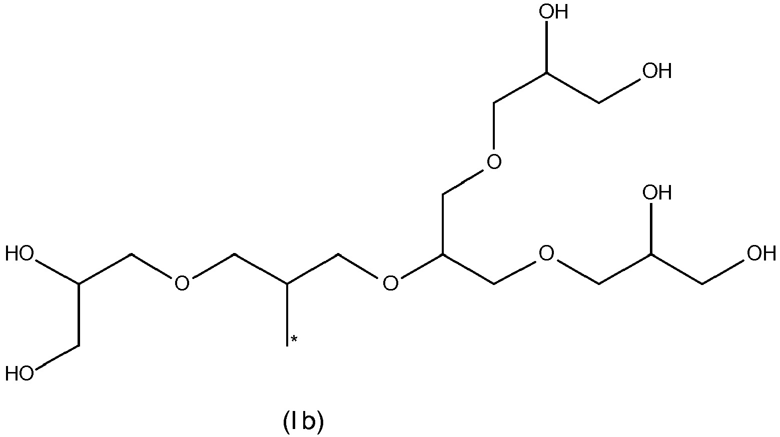

At the asterisk, the structural unit according to general formula (I), (I a) and (I b), respectively, is connected to a polymerizable group. Preferably, such polymerizable group is polymerizable by free radical polymerization, for example a carbon-carbon double bond.

The structural unit according to formula (I) and (I a) may be connected to said polymerizable group directly or through a spacer. Examples of suitable spacers are -N(R²)-, -O-, ester groups, amido groups, CH₂CH₂-O-groups, SO₂-groups, and SO₂-O-groups. R² in -N(R²)- may be selected from hydrogen, C₁-C₁₀-alkyl, C₅-C₇-cycloalkyl, benzyl and phenyl, non-substituted or up to threefold substituted with C₁-C₄-alkyl. Particularly preferred spacers are oxygen atoms, -O-, and CH₂CH₂-O-groups.

Comonomer (A) bears at least one copolymerizable group, preferably selected from vinyl, allyl, (meth)acryloyl, maleic acid and styryl groups, directly attached to group according to formula (I) or (I a) through a single bond or through a spacer.

Specific examples of comonomer (A) - in (co)polymerized form - are (A.1.1) through (A.1.5) 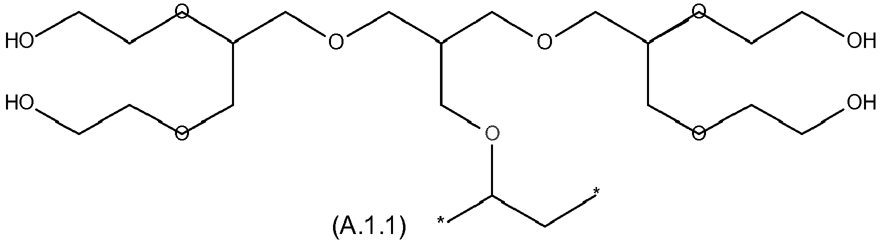 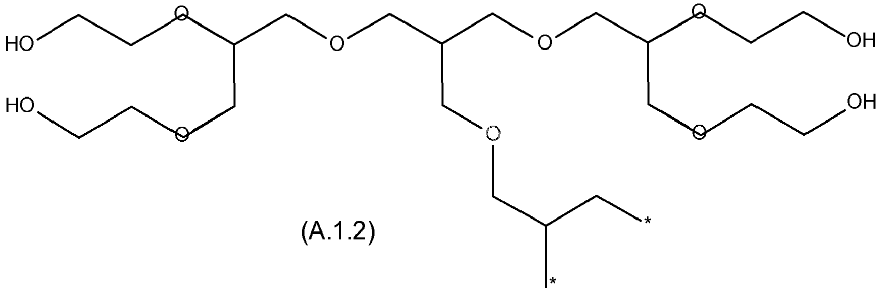 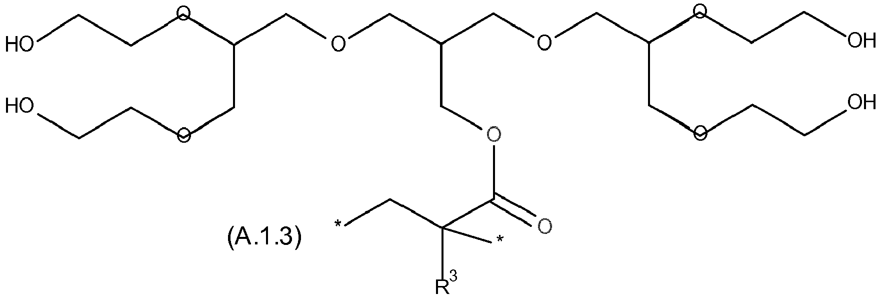 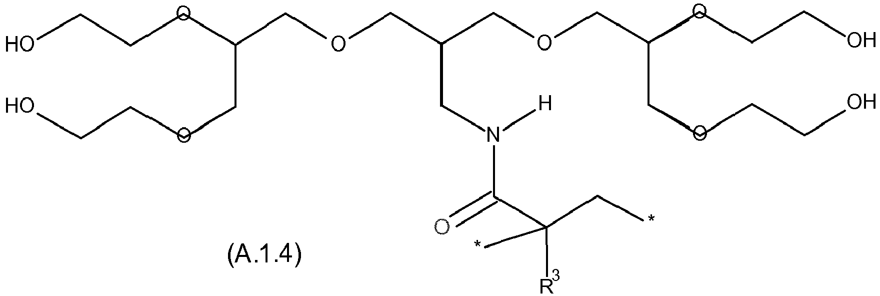 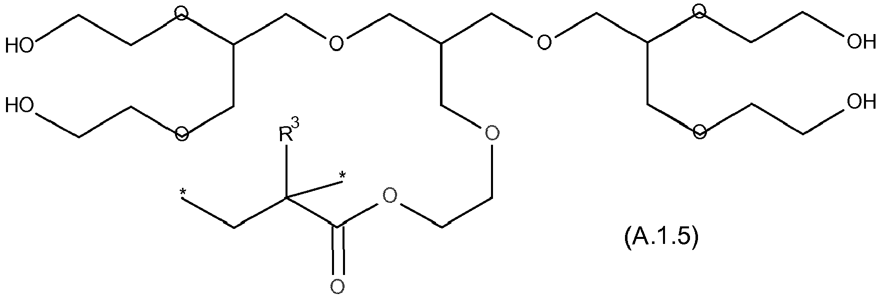 and more preferred (A.0.1) through (A.0.5)

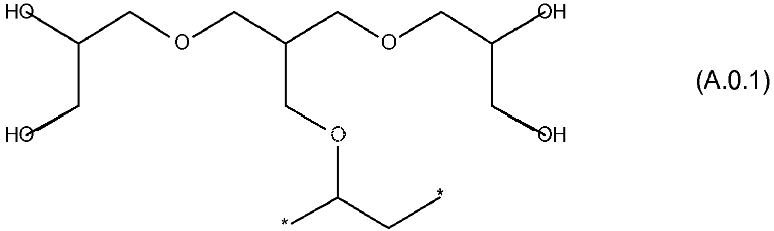

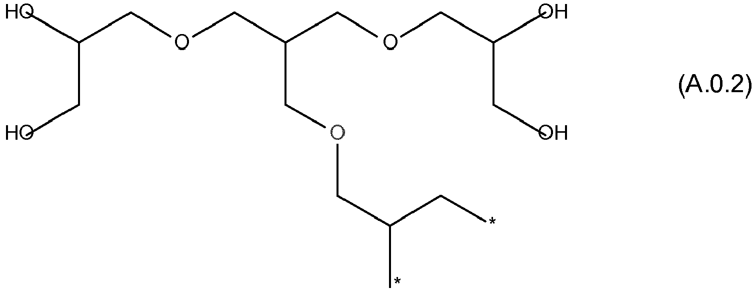

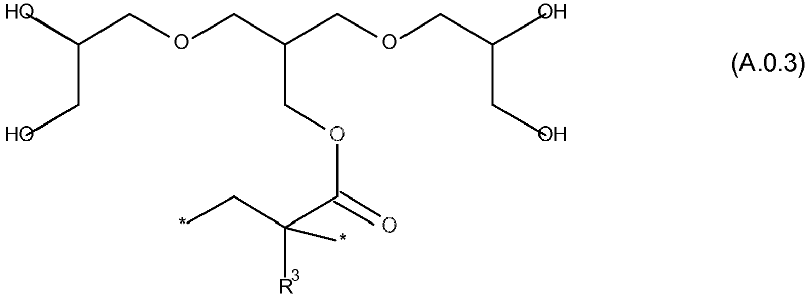

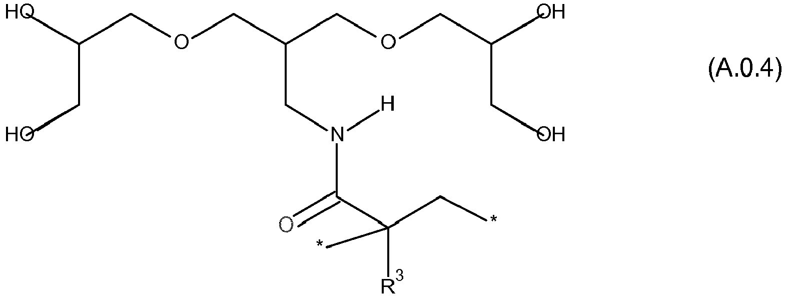

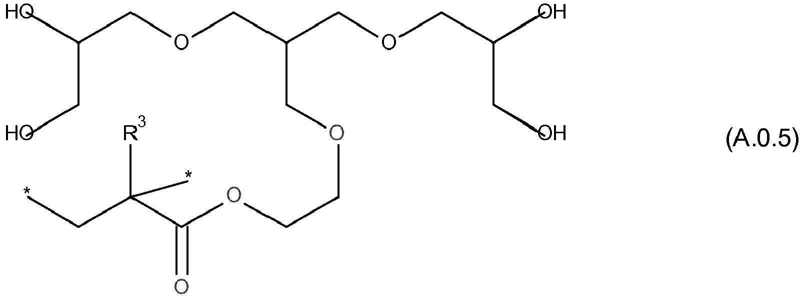

in each case R³ being identical or different and selected from hydrogen and methyl. The asterisks show where the polymer chain of (co)polymer A is continued.

Comonomer (A) may be homopolymerized to yield polymer A. In other embodiments, at least two different comonomers (A) may be copolymerized to yield copolymers A, for example (A.0.3) with (A.0.4) with R³ in both cases being hydrogen, or (A.0.3) with R³ being methyl with (A.0.4) with R³ being hydrogen.

In case (co)polymer A is a copolymer it may be selected from block copolymers, graft copolymers, and random copolymers, random copolymers being preferred.

As outlined before, in other embodiments of the present invention at least one comonomer (A) may be copolymerized with at least one comonomer (B). Comonomer (B) is selected from (meth)acrylic acid, maleic acid, maleic anhydride, fumaric acid, itaconic acid, and itaconic anhydride, with (meth)acrylic acid being preferred.

As outlined before, in other embodiments of the present invention at least one comonomer (A) may be copolymerized with at least one comonomer (C). Comonomer (C) is selected from comonomers different from both comonomers (A) and comonomers (B). Examples of comonomers (C) are styrene, α-methyl-styrene, para-methyl styrene, ethyl vinyl ether, vinyl acetate, (meth)acrylamide, and C₁-C₁₀-alkyl(meth)acrylates, for example methyl acrylate, methyl methacrylate, ethyl acrylate, ethyl methacrylate, n-butyl acrylate, n-butyl methacrylate, 2-ethylhexyl acrylate, 2-ethylhexyl methacrylate, n-decyl acrylate, n-decyl methacrylate. Further examples are 2-hydroxyethyl (meth)acrylate, 3-hydroxypropyl (meth)acrylate, N,N-dimethyl-N-ethylaminoethyl(methacrylate, counterion being selected from sulfate and halide, especially chloride, acrylamide, methacrylamide, hydroxyethyl morpholine (meth)acrylate, and hydroxyethylpyrrolidone (meth)acrylate.

In one embodiment of the present invention, (co)polymer A is composed of the following comonomers:
(A) in total 50 to 100 % by weight of comonomer(s) (A), preferably 70 to 100% by weight,
(B) in total zero to 50% by weight of comonomer(s) (B), preferably zero to 30% by weight, and
(C) in total zero to 50% by weight of comonomer(s) (C) preferably zero to 30% by weight.

In one embodiment of the present invention, (co)polymer A has an average molecular weight Mₙ in the range of from 10,000 to 5,000,000 g/mol, preferably 100,000 to 1,000,000 g/mol. The average molecular weight Mₙ may be determined, for example, by gel permeation chromatography (GPC), for example with CHCl₃ as eluent.

### (Co)polymer A is particularly suitable for the inventive coating process.

A further aspect of the present invention relates to the inventive (co)polymer synthesis. The inventive (co)polymer synthesis comprises the following steps, in the context of the present invention also referred to as steps (a), (b) and (c).

Step (a) is related to providing a comonomer (A') that bears at least one group according to general formula (II) 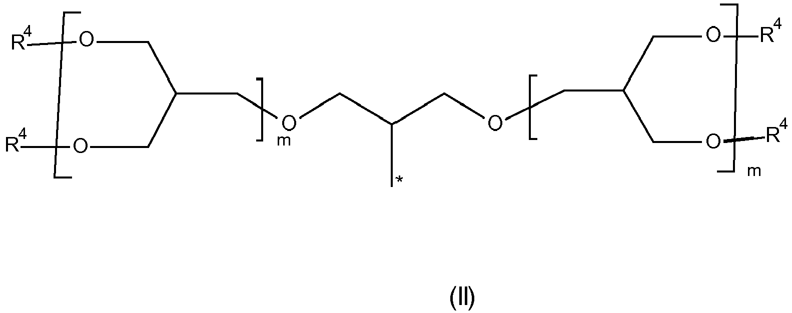 wherein
- R⁴: is

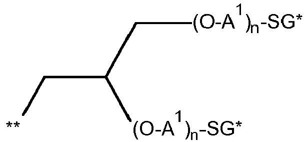

- m: is independently from each other selected from zero to 6, preferably zero to 3, and even more preferably they are identical and equal zero,
- n: is independently from each other selected from zero to 200,
- A¹: are different or identical and selected from C₂-C₁₀-alkylene, C₆-C₁₀-arylene and C₇-C₁₀-aralkylene,
- SG*: is different or identical and a protective group, or two moieties SG* form together a single protective group.

A¹ and n have been defined in more detail above.

In a particularly preferred embodiment of the present invention, each SG* is an isopropylidene group, -C(CH₃)₂-. Even more preferably, each SG* is an isopropyidene group, and all n equal 1.

In one embodiment of the present invention, comononer (A') bears a polymerizable group selected from vinyl, allyl, (meth)acryloyl, maleic acid and styryl groups. Particularly preferred are (meth)acryloyl groups.

In one embodiment of the present invention, in formula (II) n = zero, and each two groups SG* form together a -C(CH₃)₂- group.

The structural unit according to formula (II) may be connected to said polymerizable group directly or through a spacer. Examples of suitable spacers are -N(R²)-, -O-, ester groups, amido groups, CH₂CH₂-O-groups, SO₂-groups, and SO₂-O-groups. R² in -N(R²)- may be selected from hydrogen, C₁-C₁₀-alkyl, C₅-C₇-cycloalkyl, benzyl and phenyl, non-substituted or up to threefold substituted with C₁-C₄-alkyl. Particularly preferred spacers are oxygen atoms, -O-, and CH₂CH₂-O-groups.

Particularly preferred examples of comonomers are comonomers (A'.1.1) through (A'.1.5) 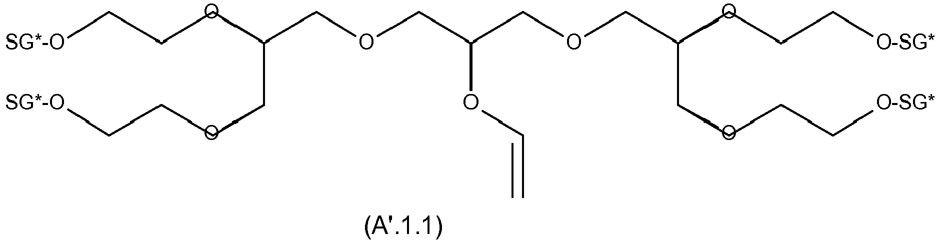 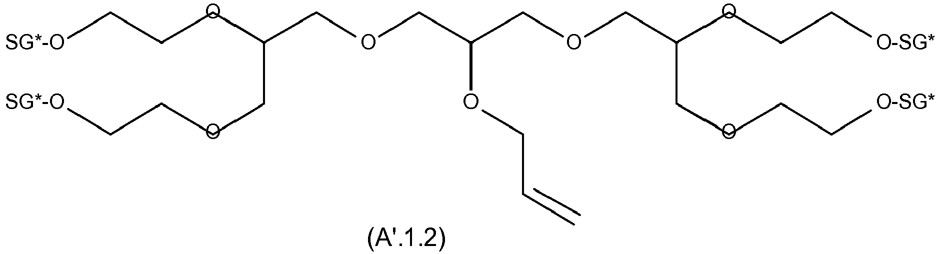 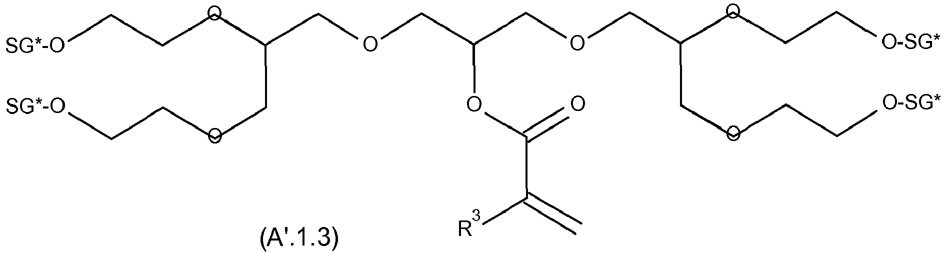 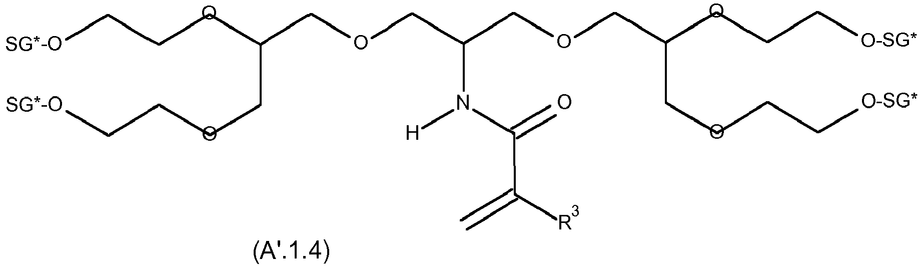 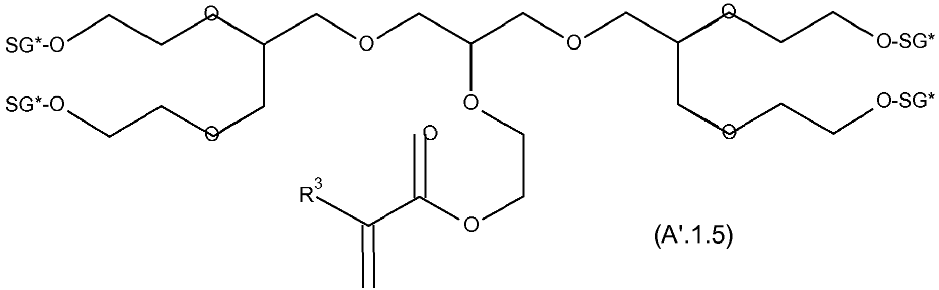 and more preferred (A'.0.1) through (A'.0.5)

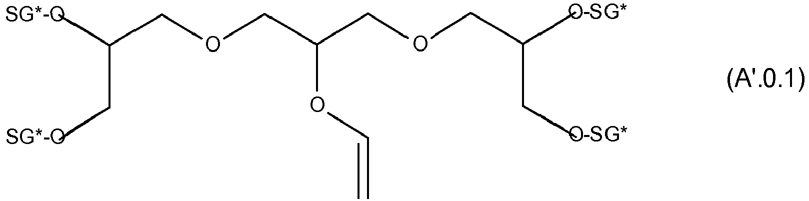

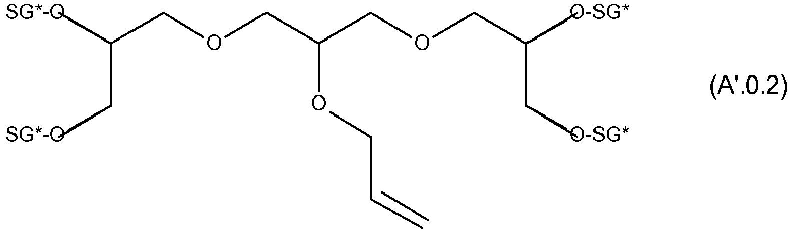

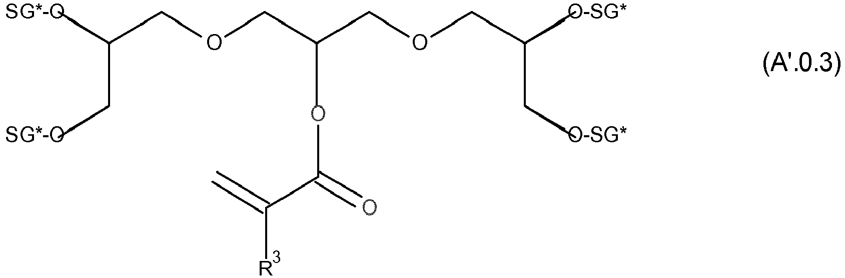

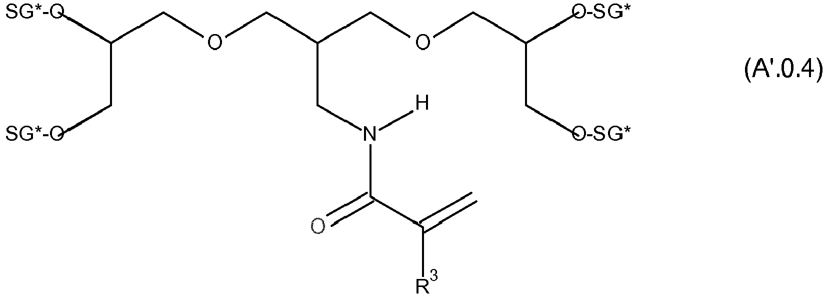

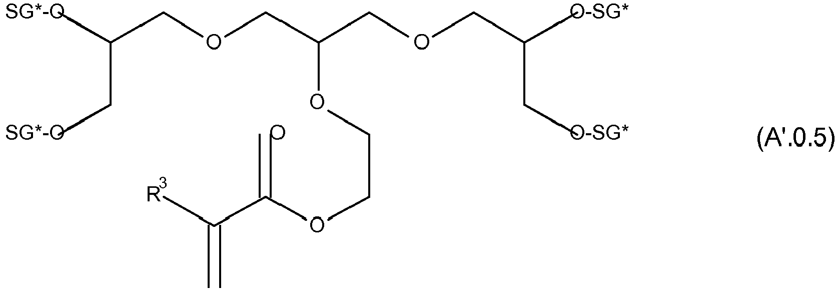

### in each case R³ being identical or different and selected from hydrogen and methyl.

In step (b), comonomer (A') is homo- or copolymerized, preferably under free-radical polymerization conditions, in bulk, in emulsion or preferably in solution. When discussing the conditions for copolymerization and homopolymerization in the context of step (b), the term "polymerization" will be used regardless whether reference is made to a homo- or copolymerization. Suitable solvents for polymerization in solution are aromatic solvents such as toluene or xylene as mixture of the isomers, chlorinated hydrocarbons such as dichloromethane or trichloromethane, N,N-dimethyl formamide (DMF) and N,N-dimethyl acetamide N-methyl-pyrrolidone (NMP), and cyclohexanone.

The polymerization is preferably initiated by at least one initiator or an initiator system. Such initiators may be selected from azo compounds and peroxides.

Examples of suitable peroxides are alkali metal peroxodisulfates, such as, for example, sodium peroxodisulfate or ammonium peroxodisulfate, hydrogen peroxide, organic peroxides, such as diacetyl peroxide, di-tert-butyl peroxide, diamyl peroxide, dioctanoyl peroxide, didecanoyl peroxide, dilauryl peroxide, dibenzoyl peroxide, bis(o-toluyl) peroxide, succinyl peroxide, tert-butyl peracetate, tert-butyl permaleate, tert-butyl perisobutyrate, tert-butyl perpivalate, tert-butyl peroctanoate, tert-butyl perneodecanoate, tert-butyl perbenzoate, di-tert-butyl peroxide, tert-butyl hydroperoxide, cumyl hydroperoxide, tert-butyl peroxy-2-ethylhexanoate and diisopropyl peroxydicarbamate. Azo compounds, such as, for example, azobisisobutyronitrile, azobis(2-amidopropane) dihydrochloride and 2,2'-azobis(2-methylbutyronitrile) are also suitable.

Redox initiators are likewise suitable, for example comprising peroxides and an oxidizable sulfur compound. Systems comprising acetone bisulfite and organic peroxide, such as tert-C₄H₉-OOH, Na₂S₂O₅ (sodium disulfite) and organic peroxide, such as tert-C₄H₉-OOH or HO-CH₂SO₂Na, and organic peroxide, such as tert-C₄H₉-OOH are very particularly preferred. Systems such as, for example, ascorbic acid/H₂O₂, are also particularly preferred.

Temperatures in the range from 20 to 105°C, preferably from 50 to 85°C, can be chosen as the polymerization temperature. The temperature chosen is dependent on the decomposition characteristics of the initiator used or of the initiators used.

The pressure conditions are generally not critical and, for example, pressures in the range from atmospheric pressure to 10 bar are suitable.

Depending on the type of polymerization it is possible to use at least one emulsifier, which may be anionic, cationic or nonionic. However, solution polymerization is preferred, and the use of no emulsifier is even more preferred.

In step (c), the protective group SG* is being removed. The reaction conditions for step (c) are selected according to the nature of the protective group SG*. In embodiments wherein SG* is an acetal or ketal - such as isopropylidene - hydrolysis under acidic conditions are preferred, such as hydrolysis with mixtures from an aqueous HCl or aqueous H₂SO₄ with THF or 1,4-dioxane or aqueous CF₃COOH solution. In embodiments wherein the protective group is a benzyloxycarbonyl group reductive cleavage is preferred.

In one embodiment of the present invention, step (c) is performed at a temperature in the range of from zero to 45°C.

By the inventive synthesis, inventive (co)polymer A may be provided readily. Of course, additional steps may be performed such as, but not limited to removal of solvent by evaporation, precipitating of (co)polymer A by addition of a precipitation agent such as a solvent that does not dissolve (co)polymer A readily, for example methanol, Diethylether and n-hexane.

The present invention is further illustrated by working examples.

### I. Synthesis of comonomers

### General remarks:

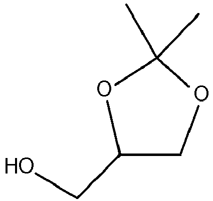

is commercially available as Solketal ® from BASF SE
DMAP: para-N,N-dimethyl aminopyridine

### 1.1 Synthesis of comonomer (A'.0.3.1)

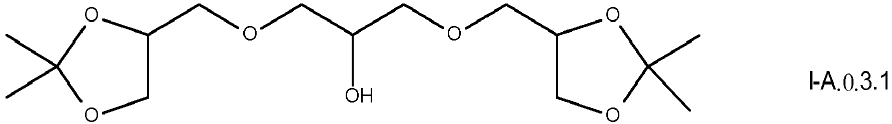

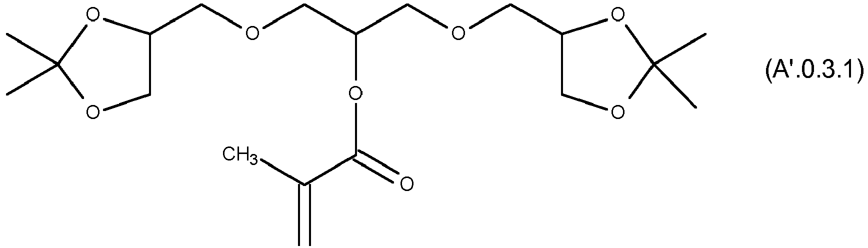

To solketal® (17.23 g, 3.0 equiv., 0.130 mol) in a three necked-flask were added KOH (4.89 g, 2.0 equiv., 0.087 mol), H₂O (3 ml) and tetrabutylammonium iodide (TBAI) (0.8 g, 5 mol%). The mixture so obtained was heated to 55°C. Epichlorohydrin (1 equiv) was added slowly. The reaction mixture was stirred at 55°C for 2 days. Then, saturated aqueous NaHCO₃ solution was added and organic materials were extracted with ethyl acetate. The combined organic phases were dried over MgSO₄ and the solvent was evaporated to afford a crude products. Unreacted solketal was distilled off as colorless oil (8.03g). The residue was purified by column chromatography to give mixture of intermediates I-A'.0.3.1 and of the respective derivative of the by-product as pale yellow oil (8.80 g). Said pale yellow oil was dissolved in dichloromethane. Diisopropylethyl amine (9.8 ml, 2.1 equiv., 56.6 mmol, "Hünig-base") and DMAP (166 mg, 0.05 equiv.) were added under N₂ atmosphere. The mixture so obtained was cooled to 0°C. Then, neat methacryloyl anhydride (8.1 ml, 2.0 equiv., 54.3 mmol) was slowly added. Thereafter the mixture was warmed to ambient temperature, thereafter to 40°C and stirred over the time of 16 hours. The obtained solution was quenched with saturated aq. NaHCO₃ and extracted with DCM. The organic phase was dried over MgSO₄ and concentrated in vacuo. The residue was purified by flash chromatography with n-hexane/ethyl acetate/ acetone (4:1:0.5, v/v) to afford (A'.0.3.1) 7.3 g, 44%) and (A'.0.3.2) (1.3 g, 11%) as pale yellow oil. MG1: ¹H-NMR (300 MHz, CDCl₃): 6.11 (s, 1H); 5.57 (t, 1.5Hz, 1H); 5.21-5.14 (m, 1H); 4.24-4.20 (m, 2H); 4.02 (d, 1.8,6.3 Hz, 2H); 3.74-3.47 (m, 10H); 1.93 (s, 3H); 1.40 (s, 6H); 1.34 (s, 6H); ¹³C-NMR (75 MHz, CDCl₃): 166.8; 136.3; 125.9; 109.5; 109.4; 74.7; 72.4; 72.3; 71.8; 71.7; 71.6; 70.2; 70.1; 66.7; 26.8; 25.5; 18.4. HRMS (ESI): 389.2172 ([M + H]⁺, C₁₉H₃₃O₈₊; calc, 389.2170 (error, -0.6 ppm)).

By-product:

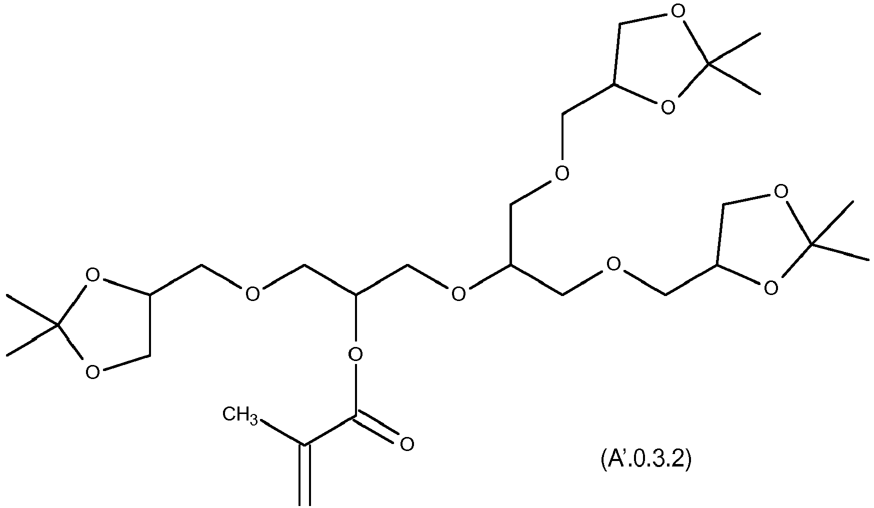

Spectroscopic data of the by-product: ¹H-NMR (300 MHz, CDCl₃): 6.11 (s, 1H); 5.57 (t, 1.5Hz, 1H); 5.15-5.12 (m, 1H); 4.27-4.18 (m, 3H); 4.05-4.00 (m, 3H); 3.78-3.43 (m, 18H); 1.94 (s, 3H); 1.40 (s, 9H); 1.35 (s, 9H). ¹³C-NMR (75 MHz, CDCl₃): 166.8; 136.3; 125.9; 109.4; 78.8; 74.7; 72.6; 72.4; 72.3; 71.7; 71.6; 70.2; 70.1; 69.0; 66.8; 26.8; 25.5; 18.4.HRMS (ESI): 594.3475 ([M + NH₄]⁺, C₂₈H₅₂NO₁₂₊; calc, 594.3484 (error, -1.5 ppm)).

### 1.2 Synthesis of comonomer (A'.0.5.1)

An amount of 0.83 g of I-A'.0.3.1 was dissolved in 4 ml DMF under an atmosphere of dry nitrogen. It was cooled to 0°C. Sodium hydride (206.4 mg, 60% in oil, 5.16 mmol, 2.0 equiv.) was added and the slurry so obtained was stirred 10 min at 0°C and 15 min at ambient temperature. 2-Benzyloxy-1-(*p*-toluenesulfonyloxy)ethane (1.58 g, 5.16 mmol) was dissolved in 2 ml dry DMF and slowly added via syringe. The reaction mixture was stirred over a period of 12 hours and then diluted with 100 ml of CH₂Cl₂. The solution was washed consecutively with water (3×30 ml), saturated aq. NaHCO₃ (30 ml) and brine (30 ml). Drying over MgSO₄ and removal of the solvent under reduced pressure afforded a crude product as brown oil. It was purified by simple flash chromatography (EA/Hexane) to give a benzoyl-protected alcohol I-A.0.3.2 as pale yellow oil (1.07 g, 91%). ¹H-NMR (300 MHz, CDCl₃): 7.34-7.26 (m, 5H); 4.55 (s, 2H); 4.28-4.20 (m, 2H); 4.04-3.99 (m, 2H); 3.80-3.45 (m, 15H); 1.41 (s, 6H); 1.35 (s, 6H).

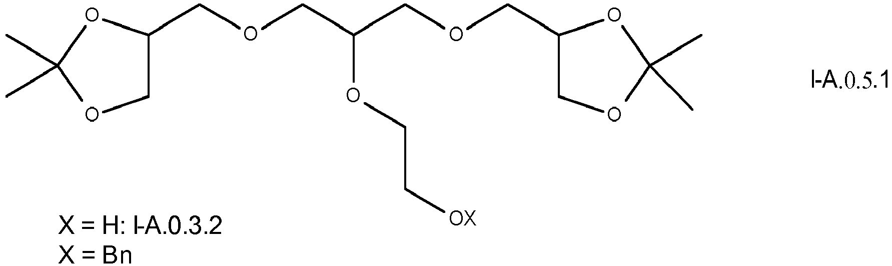

Benzoyl-protected alcohol I-A.0.3.2 (1.07 g) was dissolved in 20 mL EtOAc, to the above solution, 10% Pd-C (107 mg, 10% by weight) was added. After three vacuum / H₂ cycles to remove air from the reaction flask, then the reaction mixture was stirred over a period of 12 hours under a H₂ balloon. The mixture was filtered through celite and the solvent was removed in vacuo. Purified by simple flash chromatography (ethyl acetate/n-hexane) to give alcohol I-A.0.3.2 as a colourless oil (475 mg, 55%). ¹H-NMR (300 MHz, CDCl₃): 4.30-4.22 (m, 2H); 4.05 (dd, 6.6, 8.4 Hz, 2H); 3.76-3.47 (m, 15H); 2.99 (br., 1H); 1.41 (s, 6H); 1.35 (s, 6H).

To a solution of I-A.0.3.2 (0.47 g, 1.0 equiv., 1.29 mmol) in DCM (4.0 ml) were added Hünig base (0.47 ml, 2.1 equiv., 2.71 mmol) and DMAP (16 mg, 0.1 equiv.) under a N₂ atmosphere. The mixture was cooled to 0°C and neat methacryloyl anhydride (0.40 ml, 2.0 equiv., 2.58 mmol) was slowly added. Thereafter the reaction mixture was warmed to ambient temperature, then heated to 40°C and stirred over a period of 12 hours. The obtained solution was quenched with saturated aq. NaHCO₃ and extracted with CH₂Cl₂. The organic phase was dried over MgSO₄ and concentrated in vacuo. The residue was purified by flash chromatography with n-hexane/ethyl acetate to afford (A'.0.5.1) as colourless oil (0.47 g, 85%). ¹H-NMR (300 MHz, CDCl₃): 6.12 (s, 1 H); 5.57 (t, 1.5Hz, 1 H); 4.28-4.22 (m, 4H); 4.03 (dd, 6.3, 8.1 Hz, 2H); 3.84 (t, 4.8 Hz, 2H); 3.60-3.46 (m, 11H); 1.94 (s, 3H); 1.40 (s, 6H); 1.35 (s, 6H); ¹³C-NMR (75 MHz, CDCl₃): 167.4; 136.3; 125.7; 109.5; 78.6; 74.7; 72.6; 71.9; 71.7; 68.6; 66.8; 64.2; 26.8; 25.5; 18.4. HRMS (ESI): 450.2696 ([M + NH₄]⁺, C₂₁H₄₀NO₉⁺; calc, 450.2698 (error, -0.4 ppm)).

### II. Polymerizations

General remark: AIBN: azobis-isobutyronitrile

### II.1 Homopolymerization of comonomer (A'.0.3.1)

### II.1.1 Step (a.1)

### 1.01 mg of comonomer (A'.0.3.1) were provided in bulk.

### II.1.2: Step (b.1), polymerization

AIBN (0.1 mol%) and toluene (1.3 ml) were added to the 1.01 g of comonomer (A'.0.3.1). The solution obtained was kept at 60 °C for 17 hours and then cooled down to ambient temperature. The polymer so obtained, so-called polymerized (A'.0.3.1), was dissolved in CH₂Cl₂ and purified by precipitation with *n*-hexane. Polymerized (A'.0.3.1) was obtained as colorless sticky solid (820 mg, 81%). ¹H-NMR (300 MHz, CDCl₃): 4.25 (br., 2H); 4.04 (br., 4H); 3.57 (br., 13H); 1.76 (br., 1.52H); 1.40 (s, 6H); 1.35 (s, 6H); 0.90 (br., 2.68H); ¹³C-NMR (75 MHz, CDCl₃): 109.4; 78.2; 74.7; 72.6; 71.6; 71.5; 66.8; 27.0; 25.6. Elemental analysis (%) calcd for (C₂₁H₃₆O₉)ₙ (432.51)ₙ: C, 58.32; H, 8.39. Found: C, 58.06; H, 8.52.

### II.1.3 Step (c.1), removal of protective groups

2.5 g of polymerized (A'.0.3.1) were dissolved in 110 ml of freshly distilled 1,4-dioxane. The solution was cooled to 0°C, and 10 ml of 2N aqueous HCl were added dropwise. After the addition of the HCl had been completed the reaction mixture was allowed to warm to ambient temperature under stirring. After a total reaction time of 24 hours a solution of inventive (co)polymer 1.2 was obtained, hereinafter also referred to as inventive polymer A.1. Relevant data are summarized in Table 1.

### II.2 Polymerization of (A'.0.5.1)

### II.2.1 Step (a.2)

86 mg of comonomer (A'.0.5.1) were provided in bulk.

### II.1.2: Step (b.2), polymerization

AIBN (0.1 mol%) and toluene (0.1 ml) were added to the 86 mg of comonomer (A'.0.5.1). The solution obtained was kept at 60 °C for 15 hours and then cooled down to ambient temperature. The polymer so obtained, so-called polymerized (A'.0.5.1), was dissolved in CH₂Cl₂ and purified by precipitation with *n*-hexane. Polymerized (A'.0.5.1) was obtained as colorless sticky solid (71 mg, 82%). ¹H-NMR (300 MHz, CDCl₃): 4.25 (br., 2H); 4.04 (br., 4H); 3.57 (br., 13H); 1.76 (br., 1.52H); 1.40 (s, 6H); 1.35 (s, 6H); 0.90 (br., 2.68H); ¹³C-NMR (75 MHz, CDCl₃): 109.4; 78.2; 74.7; 72.6; 71.6; 71.5; 66.8; 27.0; 25.6. Elemental analysis (%) calcd for (C₂₁H₃₆O₉)ₙ (432.51)ₙ: C, 58.32; H, 8.39. Found: C, 58.06; H, 8.52.

### II.2.3 Step (c.2), removal of protective groups

45 g of polymerized (A'.0.5.1) were dissolved in 2 ml of freshly distilled 1,4-dioxane. The solution was cooled to 0°C, and 0.3 ml of 2N aqueous HCl were added dropwise. After the addition of the HCl had been completed the reaction mixture was allowed to warm to ambient temperature under stirring. After a total reaction time of 24 hours, a solution of inventive (co)polymer A.2 was obtained, hereinafter also referred to as inventive polymer A.2. Relevant data are summarized in Table 1.

**Table 1: data of inventive copolymers**

| Polymer | Monomer (mmol) | [AIBN] mol% | solvent (ml) | [monomer] (mol L⁻¹) | Time [h] | Yield | Mₙ [kD] | PDI | DPₙ |
|---|---|---|---|---|---|---|---|---|---|
| A.1 | (A'.0.3.1), 2.6 | 0.1 | 1.3 | 2 | 17 | 81% | 825 | 5.1 | 2100 |
| A.2 | (A'.0.5.1), 0.2 | 0.1 | 0,1 | 2 | 16 | 82% | 408 | 3.0 | 944 |

| | | | | | | | | | |
|---|---|---|---|---|---|---|---|---|---|
| PDI: polydispersity The yield refers to the yield after the respective step (b). | | | | | | | | | |

### III. Inventive coating of silicon wafers

Silicon wafers (P/B(100), Si-Mat Silicon Wafers, Germany) were cut to size, sonicated three times (10 min each) in 2-isopropanol, and dried with nitrogen gas. The polymer solution of a specific concentration was freshly prepared, into which the silicon wafer cleaned and dried as the above procedure was immersed. After the desired time, the wafer was taken out of the polymer solution and dried with nitrogen gas, then rinsed with H₂O three times and dried under a nitrogen stream. The dry thickness of the adsorbed polymers on the wafer surface was determined by VASE.

The dry thicknesses of adsorbed inventive copolymers A on the wafer surface were measured with a variable-angle spectroscopic ellipsometer (VASE, M-2000F, LOT Oriel GmbH, Darmstadt, Germany) at an incident angle of 70°, using a three-layer model (software WVASE32, LOT Oriel GmbH, Darmstadt, Germany), each sample being measured at three different locations.

## Claims

1. Process of coating silicon surfaces, comprising the step of treating said silicon surface with at least one (co)polymer A, comprising in copolymerized form
(A) at least one comonomer bearing a structural unit according to the general formula (I) 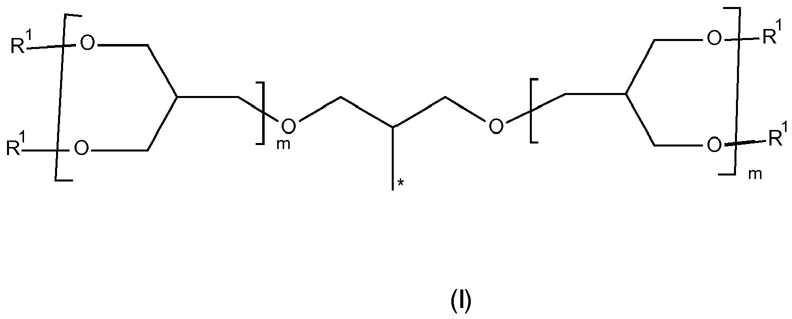 wherein
R¹ is 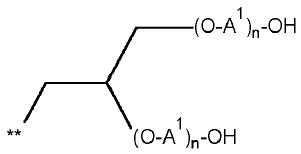
m is independently from each other selected from zero to 6,
n is independently from each other selected from zero to 200,
A¹ are different or identical and selected from C₂-C₁₀-alkylene, C₆-C₁₀-arylene and C₇-C₁₀-aralkylene,
(B) optionally, at least one ethylenically unsaturated carboxylic acid,
(C) optionally, at least one further monomer.

2. Process according to claim 1 wherein silicon surfaces are part of a silicon wafer.

3. Process according to claim 1 wherein silicon surfaces constitute a SiOx-coated polymer film, polymer being selected from PET, PE, PP, and PA.

4. Process according to any of the preceding claims wherein said surface is contacted with an aqueous solution containing at least one (co)polymer A, followed by drying.

5. Process according to any of the preceding claims wherein comononer (A) bears a polymerizable group selected from vinyl, allyl, (meth)acryloyl, maleic acid and styryl groups.

6. Silicon surface coated with at least one (co)polymer A, comprising in copolymerized form
(A) at least one comonomer bearing a structural unit according to the general formula (I) 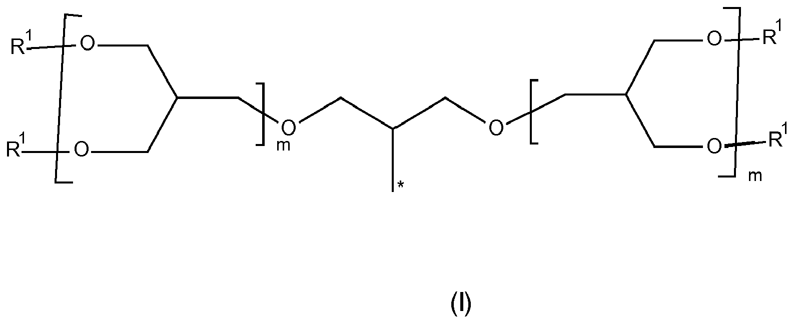 wherein
R¹ is 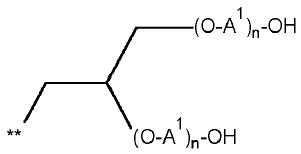
m is independently from each other selected from zero to 6,
n is independently from each other selected from zero to 200,
A¹ are different or identical and selected from C₂-C₁₀-alkylene, C₆-C₁₀-arylene and C₇-C₁₀-aralkylene,
(B) optionally, at least one ethylenically unsaturated carboxylic acid,
(C) optionally, at least one further monomer.

7. Silicon surface according to claim 6 wherein the layer of (co)polymer (A) has a thickness in the range of from 1 to 100 nm.

8. Silicon surface according to claim 6 or 7 wherein such silicon surface constitutes a part of a silicon wafer.

9. Silicon surface according to claim 6 or 7 wherein silicon surfaces constitute a SiOx-coated polymer film, polymer being selected from PET, PE, PP, and PA

10. Silicon surface according to any of the claims 6 to 9 comprising at least one further layer wherein the coating of (co)polymer (A) serves as primer.

11. (Co)polymer, comprising in copolymerized form
(A) comonomer bearing a structural unit according to the general formula (I) 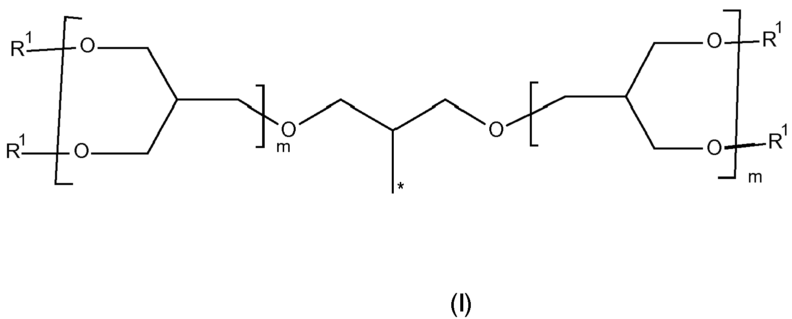 wherein
R¹ is 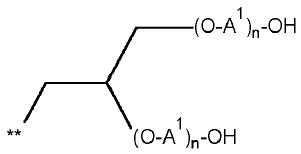
m is independently from each other selected from zero to 6,
n is independently from each other selected from zero to 200,
A¹ are different or identical and selected from C₂-C₁₀-alkylene, C₆-C₁₀-arylene and C₇-C₁₀-aralkylene,
(B) optionally, at least one ethylenically unsaturated carboxylic acid,
(C) optionally, at least one further monomer.

12. (Co)polymer according to claim 11 wherein said polymer comprises neither comonomer (B) nor (C).

13. (Co)polymer according to claim 11 or 12 wherein n = zero.

14. (Co)polymer according to any of the claims 11 to 13 wherein comononer (A) bears a polymerizable group selected from vinyl, allyl, (meth)acryloyl, maleic acid and styryl groups.

15. Process for making a (co)polymer according to any of the claims 11 to 14 comprising the following steps:
(a) Providing at least one comonomer (A') wherein comonomer (A') is a comonomer bearing a structural unit according to general formula (II) 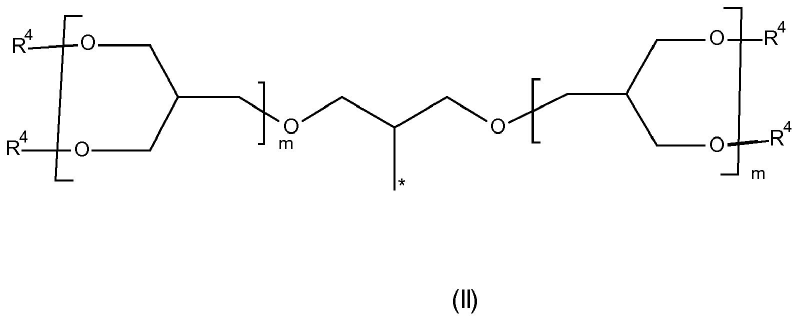 wherein
R⁴ is 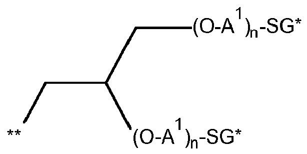
m is independently from each other selected from 1 to 6,
n is independently from each other selected from zero to 200,
A¹ are different or identical and selected from C₂-C₁₀-alkylene, C₆-C₁₀-arylene and C₇-C₁₀-aralkylene,
SG* is different or identical and a protective group, or two moieties SG* form together a single protective group,
(b) (co)polymerizing at least one comonomer (A') and, optionally, at least one comonomer (B) or at least one comonomer (C), by free-radical copolymerization,
(c) removing the protective groups SG* of the resultant (co)polymer.

16. Process according to claim 15 wherein n = zero, and each two groups SG* form together a -C(CH₃)₂- group.
